(19) **Europäisches Patentamt**
**European Patent Office**
**Office européen des brevets**

(11) **EP 1 830 783 B1**

(12) **EUROPEAN PATENT SPECIFICATION**

(45) Date of publication and mention
of the grant of the patent:
**15.08.2012  Bulletin 2012/33**

(51) Int Cl.:
***A61K 6/00*** (2006.01)

(21) Application number: **05824910.3**

(86) International application number:
**PCT/IB2005/054339**

(22) Date of filing: **20.12.2005**

(87) International publication number:
**WO 2006/067748 (29.06.2006 Gazette 2006/26)**

(54) **A DROPLET CLEANING FLUID USED FOR CLEANING TEETH WHICH INCLUDES A POLYMER ADDITIVE**

TRÖPFCHEN-REINIGUNGSFLÜSSIGKEIT ZUR REINIGUNG VON ZÄHNEN MIT EINEM POLYMERZUSATZ

LIQUIDE DE NETTOYAGE EN GOUTTES COMPRENANT UN ADDITIF POLYMERE, DESTINE AU NETTOYAGE DES DENTS

(84) Designated Contracting States:
**AT BE BG CH CY CZ DE DK EE ES FI FR GB GR HU IE IS IT LI LT LU LV MC NL PL PT RO SE SI SK TR**

(30) Priority: **21.12.2004  US 638057 P**

(43) Date of publication of application:
**12.09.2007  Bulletin 2007/37**

(73) Proprietor: **Koninklijke Philips Electronics N.V.
5621 BA Eindhoven (NL)**

(72) Inventors:
• **DUINEVELD, Paul
Briarcliff Manor, New York 10510-8001 (US)**
• **BRYANT, William E.
Briarcliff Manor, New York 10510-8001 (US)**

(74) Representative: **Damen, Daniel Martijn
Philips
Intellectual Property & Standards
P.O. Box 220
5600 AE Eindhoven (NL)**

(56) References cited:
**FR-A1- 2 696 638       GB-A- 1 328 551
US-A- 3 870 039        US-A- 5 062 795
US-B1- 6 238 648**

• **DATABASE CA CHEMICAL ABSTRACTS SERVICE, COLUMBUS, OHIO, US; XP002378637 retrieved from STN Database accession no. 126: 162016 & JP 08 333226 A (LION CORP.) 17 December 1996 (1996-12-17)**
• **DATABASE WPI Week 2004 Derwent Publications Ltd., London, GB; AN 2004-584394 XP002378639 & JP 2004 217609 A (LION CORP.) 5 August 2004 (2004-08-05)**
• **J. SOHN ET AL.: "Drag-reduction effectiveness of xanthan gum in a roating disk apparatus", CARBOHYDRATE POLYMERS, vol. 45, 2001, pages 61-68,**
• **DATABASE CA [Online] CHEMICAL ABSTRACTS SERVICE, COLUMBUS, OHIO, US Database accession no. 127:207096 & JP 9 217035 A (PILOT INK CO LTD) 19 August 1997 (1997-08-19)**

## Description

This invention relates generally to fluids used in high velocity fluid droplet systems for cleaning teeth, and more particularly concerns a system for cleaning teeth using a stream of water droplets.

U.S. application 2009/0017423 discloses an oral care system for cleaning teeth, including removing dental plaque, by the use of a spray of fine droplets directed at a selected, effective velocity against the teeth. The droplets may be generated in different ways, including forcing a liquid with high pressure through a plurality of nozzles, or by introducing fluid into a fast-flowing stream of air. Another droplet generating embodiment includes a piezoelectric element which produces a high-speed pumping effect on fluid, which accelerates the fluid through a set of nozzles.

Droplet size is important for proper cleansing action, particularly in view of practical operational limitations on flow rate and fluid velocity/pressure. If the droplets are too small, *i.e.* less than 10 μm, high velocities of up to 200 m/sec are necessary to produce a proper cleaning effect, including plaque removal. Such a high velocity is undesirable, since it dramatically increases the necessary pressure to be produced by the fluid pump, as well as increasing the energy supplied to the liquid. It has heretofore been difficult to produce droplets of desired size for good cleaning without using excessive flow rates or velocities.

Hence, it is desirable to be able to control droplet size while maintaining the droplet flow rate within acceptable limits.

Accordingly, the present invention provides a system for cleaning teeth using a stream of water droplets, comprising: a source of water, the water including a water soluble, human consumable polymer additive of selected concentration and molecular weight, namely xanthan gum, and wherein the concentration of the xanthan gum is between 0.1c*-10c*, where c* is 1/[μ] and

$$[\mu] = \lim_{c \to 0} \frac{\mu - \mu_s}{\mu_s c}$$

where c is the concentration in mass per volume of the original water, μ is the viscosity of the resulting additive solution, and $\mu_s$ is the viscosity of the original water; a power toothbrush including a fluid droplet generator for creating a stream of droplets from the water, wherein the droplets have a size in a selected range, at least 80% of the droplets being at least 10 μm in diameter; and a brushhead having nozzles for directing the stream of droplets onto a selected area of the teeth.

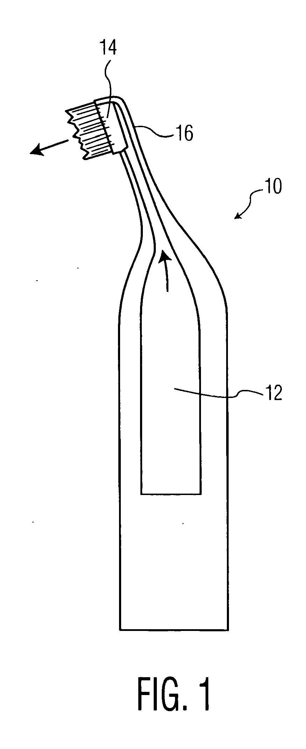

Figure 1 is a schematic view of an oral care appliance using fluid droplets.

As discussed above, U.S. patent application Serial No. 60/537,690, filed on January 20, 2004, titled "Droplet Jet System For Cleaning", which is owned by the assignee of the present invention, the contents of which is hereby incorporated by reference, is directed toward a fluid droplet spray system for cleaning teeth. As disclosed in that application, the fluid droplets may be generated by various means.

It is important for convenient operation that the flow rate of the fluid be limited. Preferably, the fluid flow rate is approximately 20 ml per brushing event, with at a flow rate of 10 ml/min for a two-minute brushing. This is a typical convenient mouth capacity for a user for a brushing event. The flow rate could be somewhat higher, however, perhaps up to 100 ml total, without significant inconvenience to the user. A range could be 5-50 ml per minute.

The desired droplet size (diameter) in such an oral care system is 10-30 μm, or more, in some cases up to 100 μm, for efficient plaque removal. In certain droplet-generating systems, the flow rate is within acceptable ranges, but the droplets are too small, while in other systems, the droplet size is within the desired range, but the flow rate is unacceptably large.

In the present invention, a small concentration of a high molecular weight polymer is added to the fluid, i.e. water, prior to the generation of the droplets, to produce what is referred to herein as an additive solution. The polymer helps in the production of a desired droplet size. The droplets can then be applied with a destined flow rate to achieve convenient, effective cleaning. The additive solution can be used with any droplet-generating system. The high molecular weight polymers can also be added to oral care fluids other than water, such as for instance, mouthwashes.

The added polymer must be water-soluble, and must be consumable, *i.e.* safe for human consumption. The possible polymer is xanthan gum. Both the molecular weight of the selected polymer and the concentration of the polymer in the fluid of origin are important. The concentration of the high molecular weight polymer should be relatively small so as to limit any increase of shear viscosity of the resulting additive solution. This is discussed in more detail below. The molecular weight of the polymer must be high enough that even at desired small concentrations, the elongational viscosity of the resulting additive solution increases significantly relative to the fluid of origin. The elongational viscosity is the resistance of the fluid to an elongational flow.

For instance, any Newtonian fluid of origin such as water will have an elongational viscosity of three times its shear viscosity. This results in droplets which break up sufficiently easily during formation that the resulting droplets are too small for oral care systems. When a small concentration of a high molecular weight polymer is added to the liquid of origin, the liquid becomes non-Newtonian, resulting in, among other effects, an increase in the elongational viscosity to values much greater than three times the shear viscosity. This increased elongational viscosity refers to the characteristic of a fluid which maintains a droplet configuration of a particular size without separating into smaller droplets. Basically, with a high elongational viscosity, existing droplets "stretch" considerably before separating into smaller droplets. A high elongational viscosity thus provides the desired result of maintaining larger droplet sizes during droplet generation. It has been discovered that the molecular weight of the added polymer should be sufficiently high that the elongational viscosity of the additive solution be approximately six or more times the shear viscosity of the additive solution to produce the desired droplet size.

The effect of the high molecular weight polymer, as indicated above, on the fluid of origin is to decrease the tendency of the resulting additive solution to break up into small droplets, i.e. the size of droplets which would be otherwise produced during droplet generation. This is accomplished by the increase in elongational viscosity of the additive solution accompanied by only a relatively small increase in shear viscosity, due to the small concentration of the high molecular weight polymer additive.

In addition, the high molecular weight polymer prevents the formation of satellite droplets during droplet generation, and further, decreases the drag of the fluid during turbulent flow, which occurs in one or more of the droplet generation systems described in the 60/537,690 application, particularly those embodiments using nozzles.

Xanthan gum has a molecular weight of approximately $7.6 \times 10^6$ g/mol. The minimum molecular weight for this application is on the order of $0.1 \times 10^6$ g/mol. Xanthan gum will produce the desired result. The molecular weight of the additive polymer, as explained above, must be sufficiently high as to substantially increase the elongational viscosity of the resulting additive solution, with the concentration of the additive polymer being quite small such that any increase in shear viscosity of the additive solution is also small. The droplets produced are substantially all (at least 80%) approximately at least 10 micrometers in diameter. Typically, the percentage is much closer to 100%.

A particular term "intrinsic" viscosity $[\mu]$ of a solution is defined herein as:

$$[\mu] = \lim_{c \longrightarrow 0} \frac{\mu - \mu_s}{\mu_s c}$$

where c is the concentration in mass per unit volume of original fluid, $\mu$ is the viscosity of the additive solution and $\mu_s$ is the viscosity of the original fluid. At a concentration c* which is larger than $1/[\mu]$), the shear viscosity of the additive solution will be significantly influenced, which can be undesirable.

For the additive polymer to be effective, the concentration should be between 0.1c*-10c*, but more preferably is in the range 0.25c*-2.5c*.

For xanthan gum and water, c* = 0.0045% weight has been found to be effective.

A cleaning system involving the above-described fluid includes a power toothbrush, such as shown in Figure 1, generally at 10. The toothbrush includes a droplet-generating system, shown generally at 12. The fluid containing the polymer additive in operation is forced out through nozzles 14 in the brushhead 16. The fluid coming out of the brushhead breaks up into a fine spray, at a desired flow rate and velocity, as well as with a desired droplet size controlled to an extent by the high molecular weight polymer additive. The spray is directed to the teeth, resulting in cleaning of the teeth, in particular plaque removal.

## Claims

1. A system for cleaning teeth using a stream of water droplets, comprising:

   a source of water, the water including a water-soluble human-consumable polymer additive of selected concentration and molecular weight, namely xanthan gum, and wherein the concentration of the xanthan gum is between 0.1c*-10c*, where c* is $1/[\mu]$ and

$$[\mu] = \lim_{c \to 0} \frac{\mu - \mu_s}{\mu_s c}$$

   where c is the concentration in mass per volume of the original water, $\mu$ is the viscosity of the resulting additive solution, and $\mu_s$ is the viscosity of the original water;
   a power toothbrush (10) including a fluid droplet generator (12) for creating a stream of droplets from the water, wherein the droplets have a size in a selected range, at least 80% of the droplets being at least 10 micrometers in diameter; and
   a brushhead (16) having nozzles (14) for directing the stream of droplets onto a selected area of the teeth.

2. The system of claim 1, wherein the flow rate of the water is within the range of 5-50 milliliters per minute.

3. The system of claim 1, wherein the droplets are within the range of 10-100 micrometers in diameter.

## Patentansprüche

1. System zur Reinigung von Zähnen unter Verwendung eines Stroms von Wassertropfen, mit:

einer Wasserquelle, wobei das Wasser einen wasserlöslichen, für den menschlichen Verzehr geeigneten Polymerzusatz in ausgewählter Konzentration und mit ausgewähltem Molekulargewicht, und zwar Xanthan, enthält, und wobei die Konzentration des Xanthans zwischen 0,1c* und 10c* liegt, wobei c* = 1/[μ] und

$$[\mu] = \frac{\lim}{c \to 0} \frac{\mu - \mu_s}{\mu_s c}$$

wobei c die Massenkonzentration pro Volumen des ursprünglichen Wassers, μ die Viskosität der sich ergebenen Zusatzlösung und μ_s die Viskosität des ursprünglichen Wassers darstellen;

einer elektrischen Zahnbürste (10) mit einem Fluidtropfengenerator (12) zur Erzeugung eines Stroms von Wassertropfen, wobei die Tropfen eine Größe in einem ausgewählten Bereich aufweisen, wobei mindestens 80% der Tropfen mindestens 10 Mikrometer im Durchmesser betragen; sowie

einem Bürstenkopf (16) mit Düsen (14), um den Strom von Tropfen auf eine ausgewählte Fläche der Zähne zu richten.

2. System nach Anspruch 1, wobei die Durchflussmenge des Wassers im Bereich von 5-50 Milliliter pro Minute liegt.

3. System nach Anspruch 1, wobei die Tropfen im Bereich von 10-100 Mikrometer im Durchmesser liegen.

**Revendications**

1. Système pour nettoyer les dents en utilisant un courant de gouttelettes d'eau, comprenant :

une source d'eau, l'eau comprenant un additif polymère hydrosoluble à consommation humaine de concentration et poids moléculaire sélectionnés, à savoir de la gomme de xanthane, et dans lequel la concentration en gomme de xanthane est entre 0,1c* et 10c*, où c* est 1/[μ] et

$$[\mu] = \lim_{c \to 0} \frac{\mu - \mu_S}{\mu_S c}$$

où c est la concentration, en masse par volume, en eau à l'origine, μ est la viscosité de la solution additive résultante, et μ_s est la viscosité de l'eau à l'origine ;

une brosse à dents électrique (10) comprenant un générateur de gouttelettes de fluide (12) pour créer un courant de gouttelettes à partir de l'eau, dans lequel les gouttelettes possèdent une taille dans une plage sélectionnée, au moins 80 % des gouttelettes mesurant au moins 10 micromètres de diamètre ; et

une tête de brosse (16) possédant des buses (14) pour diriger le courant de gouttelettes sur la zone sélectionnée des dents.

2. Système selon la revendication 1, dans lequel le débit de l'eau est dans la plage de 5 à 50 millilitres par minute.

3. Système selon la revendication 1, dans lequel les gouttelettes sont dans la plage de 10 à 100 micromètres de diamètre.

14

16

10

12

FIG. 1

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20090017423 A

- US 53769004 P